# EUROPÄISCHE PATENTSCHRIFT

(11) **EP 1 207 868 B1**
(45) Veröffentlichungstag und Bekanntmachung des Hinweises auf die Patenterteilung: **11.04.2007**
(21) Anmeldenummer: 00958375.8
(22) Anmeldetag: 03.08.2000
(51) Int. Cl.: A61K 31/135, A61P 1/04, A61P 1/12, A61P 9/00, A61P 11/00, A61P 13/00, A61P 25/08, A61P 25/24, A61P 25/00, A61P 25/30, A61P 29/00, A61P 37/08

(54) **PHARMAZEUTISCHE TRAMADOLSALZE**
PHARMACEUTICAL TRAMADOL SALTS
SELS PHARMACEUTIQUES DE TRAMADOL

(30) Priorität: 31.08.1999 DE 19940740
(43) Veröffentlichungstag der Anmeldung: 29.05.2002
(73) Patentinhaber: Grünenthal GmbH, 52078 Aachen (DE)
(72) Erfinder: KUGELMANN, Heinrich, 52068 Aachen (DE)
(86) Internationale Anmeldenummer: PCT/EP2000/007526
(87) Internationale Veröffentlichungsnummer: WO 2001/015682

(56) Entgegenhaltungen:
- EP-A- 0 144 960
- WO-A-00/12067

## Beschreibung

Die vorliegende Erfindung betrifft pharmazeutische Salze aus dem Wirkstoff Tramadol und wenigstens einem Zuckeraustauschstoff, Arzneimittel enthaltend diese Salze, die Verwendung dieser Salze zur Herstellung von Arzneimitteln sowie Darreichungsformen enthaltend diese Salze.

Tramadolhydrochlorid - (1RS,2RS)-2-[(Dimethylamino)methyl]-1-(3-methoxyphenyl)-cyclohexanol-Hydrochlorid - eignet sich zur Bekämpfung starker und mittelstarker Schmerzen und zur Behandlung von Harninkontinenz. Die US-PS-3,652,589 und die WO98/46216 beschreiben die Verwendung weiterer Salze des Wirkstoffes Tramadol mit anorganischen Säuren, wie z.B. Schwefelsäure, Salpetersäure oder Phosphorsäure, und mit organischen Säuren, wie z.B. Benzoesäure, Salicylsäure oder Phthalsäure, zur Herstellung eines Arzneimittels zur Bekämpfung von Schmerzen bzw. zur Behandlung von Harninkontinenz.

Trotz der ausgezeichneten Wirksamkeit der genannten Salze bei der Schmerzbekämpfung führt der stark bittere Geschmack des Wirkstoffes Tramadol und seiner leicht löslichen Salze bei Patienten zu einer mangelnden Einhaltung der Dosierungsvorschrift und zu mangelnder Akzeptanz der Arzneimittel, die diesen Wirkstoff schon bei der Einnahme freisetzen. Umhüllungs- und Komplexierungsverfahren, wie z.B. das Aufbringen von Filmüberzügen, die einer Verbesserung des Geschmacks dienen, beeinträchtigen dagegen die sofortige Freisetzung des Wirkstoffes aus der Arzneiform.

Die Herstellung retardierter Arzneiformen ist aufgrund der sehr guten Wasserlöslichkeit des Tramadolhydrochlorids ebenfalls erschwert und macht den Einsatz aufwendiger Retardierungsverfahren, wie z.B. das Aufbringen retardierender Überzüge oder das Einbetten des Wirkstoffes in eine retardierende Matrix, erforderlich.

Aufgabe der vorliegenden Erfindung war es daher, pharmazeutische Verbindungen für den Wirkstoff Tramadol zur Verfügung zu stellen, die den bitteren Geschmack des Tramadols nicht aufweisen und die Tramadol im Vergleich zu dem herkömmlichen, meist verwendeten Salz, nämlich Tramadolhydrochlorid, effektiver retardieren können.

Erfindungsgemäß wird diese Aufgabe durch die Bereitstellung pharmazeutischer Salze aus dem Wirkstoff Tramadol und wenigstens einem Zuckeraustauschstoff gelöst.

Vorzugsweise beträgt die Löslichkeit dieser Salze in Wasser und/oder in wäßrigen Flüssigkeiten ≤ 100 mg/ml, besonders bevorzugt ≤ 30 mg/ml und ganz besonders bevorzugt ≤ 10 mg/ml.

Als Zuckeraustauschstoffe sind alle aciden Zuckeraustauschstoffe geeignet, die unter Ausbildung einer wenigstens einfach negativ geladenen Form mit dem Wirkstoff Tramadol ein Salz bilden können. Vorzugsweise ist der Zuckeraustauschstoff Saccharin, Cyclamat, Acesulfam oder ein Gemisch aus wenigstens zwei dieser Zuckeraustauschstoffe.

Zur Herstellung der erfindungsgemäßen pharmazeutischen Salze wird Tramadol und/oder wenigstens ein sehr gut wasserlösliches Salz des Tramadols mit wenigstens einer freien Säure und/oder wenigstens einem wasserlöslichen Salz wenigstens eines Zuckeraustauschstoffes umgesetzt. Vorzugsweise wird ein solches Salz des Tramadols mit dem wasserlöslichen Salz des Zuckeraustauschstoffes in wäßrigem Medium bei neutralem pH-Wert umgesetzt. Als Salz des Tramadols wird bevorzugt Tramadolhydrochlorid eingesetzt. Als Salz des Zuckeraustauschstoffes wird bevorzugt das Natrium-, Kalium-, Calcium oder Ammoniumsalz von Saccharin und/oder Cyclamat und/oder Acesulfam und/oder als freie Säure des Zuckeraustauschstoffes die freie Säure von Saccharin und/oder Cyclamat und/oder Acesulfam eingesetzt. Sofern Tramadol an sich mit der freien Säure eines Zuckeraustauschstoffes umgesetzt wird, werden diese in einem organischen Lösungsmittel, vorzugsweise in Alkanolen und besonders bevorzugt in Ethanol umgesetzt.

Ein weiterer Gegenstand der Erfindung sind Arzneimittel, die als pharmazeutischen Wirkstoff wenigstens ein erfindungsgemäßes Tramadol-Salz und gegebenenfalls weitere Wirkstoffe und/oder Hilfsstoffe enthalten. Vorzugsweise werden die Arzneimittel zur Bekämpfung/Behandlung von/bei Schmerzen, Harninkontinenz, Husten, inflammatorischen und allergischen Reaktionen, Depressionen, Drogen- und/oder Alkoholmißbrauch, Gastritis, Diarrhoe, cardiovaskulären Erkrankungen, Atemwegserkrankungen, seelischen Erkrankungen und/oder Epilepsie eingesetzt.

Gegenstand der Erfindung ist auch die Verwendung wenigstens eines erfindungsgemäßen Tramadol-Salzes zur Herstellung eines Arzneimittels zur Bekämpfung/Behandlung von/bei Schmerzen, Harninkontinenz, Husten, inflammatorischen und allergischen Reaktionen, Depressionen, Drogen- und/oder Alkoholmißbrauch, Gastritis, Diarrhoe, cardiovaskulären Erkrankungen, Atemwegserkrankungen, seelischen Erkrankungen und/oder Epilepsie.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise wird wenigstens ein erfindungsgemäßes Tramadol-Salz in Mengen appliziert, deren Gehalt an dem Wirkstoff Tramadol 1 bis 600 mg/Tag entspricht.

Ein weiterer Gegenstand der Erfindung sind auch Darreichungsformen enthaltend wenigstens ein erfindungsgemäßes Tramadol-Salz.

Die Mengen des Tramadols und des Zuckeraustauschstoffes in den erfindungsgemäßen Arzneimittel- bzw. Darreichungsformen sind so zu wählen, daß der bittere Geschmack des Wirkstoffes Tramadol durch den Geschmack des Zuckeraustauschstoffes kompensiert wird. Vorzugsweise enthalten die erfindungsgemäßen Darreichungsformen den Zuckeraustauschstoff in einer äquimolaren Menge zum Tramadol, d.h. die beiden Komponenten liegen praktisch komplett als Salz vor. In Abhängigkeit von der Süße des eingesetzten Zuckeraustauschstoffes und/oder dem gewünschten Freisetzungsprofil für das Tramadol können die erfindungsgemäßen Darreichungsformen das Tramadol und den Zuckeraustauschstoff auch in unterschiedlichen molaren Mengen enthalten.

Bei den erfindungsgemäßen Darreichungsformen kann es sich um feste, halbfeste oder flüssige, vorzugsweise orale Arzneimittelformulierungen handeln, die neben dem Salz aus dem Wirkstoff Tramadol und aus wenigstens einem Zuckeraustauschstoff gegebenenfalls weitere Wirkstoffe sowie die üblichen Hilfs- und Zusatzstoffe enthalten.

Vorzugsweise liegen die festen erfindungsgemäßen Darreichungsformen multipartikulär, besonders bevorzugt als Granulate, Mikropartikel, Mikrotabletten oder Pellets und gegebenenfalls in Kapseln abgefüllt oder als Tabletten, vorzugsweise schnell zerfallende Tabletten oder Brausetabletten, wobei die Tabletten vorzugsweise aus Pellets verpreßt oder durch Schmelzextrusion hergestellt wurden, formuliert vor.

Sofern die festen erfindungsgemäßen Darreichungsformen zur Freisetzung der Wirkstoffe über den Darmtrakt vorgesehen sind, müssen sie wenigstens einen magensaftresistenten Überzug aufweisen, der sich pH-abhängig auflöst. Durch diesen Überzug wird erreicht, daß sie den Magentrakt unaufgelöst passieren und der/die Wirkstoff(e) erst im Darmtrakt zur Freisetzung gelangt/gelangen.

Ebenfalls bevorzugt liegen die erfindungsgemäßen Darreichungsformen als Gel, Kaugummi, Saft, besonders bevorzugt als öliger oder wäßriger Saft, oder als Spray, besonders bevorzugt als Sublingualspray, formuliert vor. Eine bevorzugte Ausführungsform der erfindungsgemäßen Darreichungsformen, zur Abgabe des Tramadols über den Magentrakt sind z. B. ölige oder wäßrige Säfte, die den oder die Wirkstoff(e) ohne Verzögerung freisetzen.

Eine Retardierung und somit auch eine weitere Modifizierung der Freisetzung des Wirkstoffes Tramadol und gegebenenfalls weiterer Wirkstoffe kann durch das Aufbringen wenigstens eines retardierenden Überzuges, die Einbettung des Wirkstoffsalzes in wenigstens eine retardierende Matrix oder durch eine Kombination daraus erfolgen.

Vorzugsweise wird eine Retardierung mit Hilfe von retardierenden Überzügen erreicht. Geeignete, retardierende Überzüge umfassen wasserunlösliche Wachse oder Polymere, wie z.B. Acrylharze, vorzugsweise Poly(meth)acrylate, oder wasserunlösliche Cellulosen, vorzugsweise Ethylcellulose. Diese Materialien sind aus dem Stande der Technik, z.B. Bauer, Lehmann, Osterwald, Rothgang "Überzogene Arzneiformen", Wissenschaftliche Verlagsgesellschaft mbH Stuttgart, 1988, Seite 69 ff., bekannt.

Neben den wasserunlöslichen Polymeren können zur Einstellung der Freisetzungsgeschwindigkeit des Wirkstoffes die Retardüberzüge gegebenenfalls auch nicht retardierende, vorzugsweise wasserlösliche Polymere in Mengen bis zu 30 Gew.%, wie Polyvinylpyrrolidon oder wasserlösliche Cellulosen, vorzugsweise Hydroxypropylmethylcellulose oder Hydroxypropylcellulose, und/oder hydrophile Porenbildner, wie Saccharose, Natriumchlorid oder Mannitol und/oder die bekannten Weichmacher enthalten.

Ebenfalls bevorzugt können die erfindungsgemäßen Darreichungsformen zur Retardierung des Wirkstoff-Salzes dieses auch in einer retardierenden Matrix, vorzugsweise gleichmäßig verteilt, enthalten.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophobe Polymere, Wachse, Fette, langkettigen Fettsäuren, Fettalkohole oder entsprechenden Ester oder Ether oder deren Gemische. Besonders bevorzugt werden als hydrophobe Materialien Mono- oder Diglyceride von C₁₂-C₃₀-Fettsäuren und/oder C₁₂-C₃₀-Fettalkohole und/oder Wachse oder deren Gemische eingesetzt.

Es ist auch möglich, Mischungen der genannten hydrophilen und hydrophoben Materialien als retardierendes Matrixmaterial einzusetzen.

Die Herstellung der erfindungsgemäßen Darreichungsformen kann nach den verschiedenen dem Fachmann bekannten Methoden erfolgen. Diese Methoden sind aus dem Stande der Technik, z.B. "Pharmaceutical Pelletization Technology", Drugs and the Pharmaceutical Sciences Vol 37, Verlag Marcel Decker oder "Remington's Pharmaceutical Sciences", Mack Publishing Company, Easton, Pennsylvania bekannt.

Sofern die erfindungsgemäßen Darreichungsformen, wie z.B. die Tabletten oder die Pellets Überzüge aufweisen, können diese nach üblichen Verfahren, wie z.B. Dragieren, Aufsprühen von Lösungen, Dispersionen oder Suspensionen, durch Schmelzverfahren oder durch Pulverauftragsverfahren aufgebracht werden.

Aus erfindungsgemäßen Darreichungsformen, die zur Abgabe des Tramadols über die Mundschleimhaut eingesetzt werden, wie z. B. aus Gel, Kaugummi oder aus Sublingualspray, wird eine weitgehend konstante Abgabe des Tramadols ohne den Einsatz einer retardierenden Matrix und/oder eines retardierenden Überzuges erreicht.
Aus erfindungsgemäßen Darreichungsformen, die zur Abgabe des Tramadols über den Darmtrakt eingesetzt werden, wie z. B. aus Kapseln, Tabletten, Granulaten oder Pellets wird eine konstante Abgabe des Tramadols ebenfalls ohne den Einsatz einer retardierenden Matrix und/oder eines retardierenden Überzuges, aber mit einem magensaftresistenten Überzug versehen, erreicht.

Die erfindungsgemäßen Darreichungsformen haben weiterhin den Vorteil, daß der stark bittere Geschmack des Tramadols durch die gleichzeitige Freisetzung eines Zuckeraustauschstoffes kompensiert wird. Hierdurch verbessert sich die Einhaltung der Dosierungsvorschrift bei den Patienten und die Arzneimittel, die den Wirkstoff Tramadol enthalten, erfahren eine größere Akzeptanz. Die erfindungsgemäßen Arzneimittel sind auch für Diabetiker geeignet. Die Bildung eines Salzes aus Tramadol und einem Zuckeraustauschstoff und/oder einem Hilfsstoff mit einer Löslichkeit von ≤ 100 mg/ml in Wasser und/oder wäßrigen Körperflüssigkeiten ermöglicht eine im Vergleich zu Tramadolhydrochlorid effektivere Retardierung des Wirkstoffes Tramadol mit üblichen Retardierungsverfahren. Retardierte Arzneimittel, die diese erfindungsgemäßen Tramadol-Salze enthalten, können daher einfacher und kostengünstiger produziert werden. Dies gilt auch für andere Modifizierungen der erfindungsgemäßen Arzneimittel wie z. B. mit magensaftresistenten Überzügen.

Die Löslichkeit der Salze aus Tramadol und einem Zuckeraustauschstoff wurde wie folgt bestimmt:
Von dem Salz aus (1RS,2RS)-2-[(Dimethylamino)methyl]-1-(3-methoxyphenyl)-cyclohexanol und dem entsprechenden Zuckeraustauschstoff wurde bei 25 °C eine so große Menge in ionenfreies Wasser gegeben (z.B. für Tramadolsaccharinat ca. 1 g auf 10 ml ionenfreies Wasser), daß eine bei dieser Temperatur gesättigte Lösung entstand, die auch noch nach 20 Stunden rühren bei 25 °C gesättigt war. Die hierzu erforderliche Menge des Salzes aus Tramadol und dem entsprechenden Zuckeraustauschstoff kann ggf. durch Vorversuche ermittelt werden.
   Nach dem Absetzen lassen des nicht gelösten Salzes aus Tramadol und dem entsprechenden Zuckeraustauschstoff wurde der klare Überstand abpipettiert und bei mindestens 3000 Umdrehungen pro Minute für 5 Minuten zentrifugiert. Ein Teil des so erhaltenen klaren Überstandes wird in ein HPLC-Probenfläschen überführt und die Konzentration des Salzes aus Tramadol und dem entsprechenden Zuckeraustauschstoff wird gegen Tramadolhydrochlorid als Standard bestimmt.
Im folgenden wird die Erfindung anhand der Beispiele erläutert. Die Erläuterungen sind lediglich beispielhaft und schränken den allgemeinen Erfindungsgedanken nicht ein.

### Beispiel 1:

Zur Herstellung von Tramadolsaccharinat wurden 30,0 g (0,1 mol) Tramadolhydrochlorid und 20,53 g (0,1 mol) Saccharin-Natrium oder 24,13 g Saccharin-Natrium-Dihydrat (0,1 mol) jeweils unter Erwärmen in einer möglichst geringen Menge Wasser vollständig gelöst. Anschließend wurden beide Lösungen unter Rühren miteinander vermischt. Das Tramadolsaccharinat kristallisierte beim Abkühlen aus der wäßrigen Lösung schon nach kurzer Zeit aus und wurde nach herkömmlichen Methoden isoliert und mit Ethanol gereinigt.

Die Löslichkeit des so erhaltenen Tramadolsaccharinates in Wasser wurde nach obenstehend angegebener Methode bestimmt und beträgt 22,5 mg/ml.

### Beispiel 2:

30,0 g (0,1 mol) Tramadolhydrochlorid wurden in 20 g Wasser gelöst und unter Rühren langsam mit einer Lösung von 20,13 g (0,1 mol) Natriumcyclamat in 36 g Wasser vermischt. Die resultierende Lösung wurde anschließend für 16 Stunden bei einer Temperatur von 5°C gelagert. Das Tramadolcyclamat wurde in kristalliner Form erhalten, nach herkömmlichen Methoden isoliert und mit Ethanol gereinigt.

### Beispiel 3:

30,0 g (0,1 mol) Tramadolhydrochlorid wurden in 13 g Wasser gelöst und unter Rühren langsam mit einer Lösung von 20,13 g (0,1 mol) Acesulfam-Kalium in 53 g Wasser vermischt. Die resultierende Lösung wurde anschließend über Nacht bei 5 °C gelagert. Das Tramadolacesulfamat wurde in kristalliner Form erhalten, nach herkömmlichen Methoden isoliert und mit Ethanol gereinigt.

### Beispiel 4:

Zur Herstellung eines oralen Gels wurden zunächst 0,33 g Methylparaben, 0,05 g Propylparaben und 75,0 g Xylitol bei einer Temperatur von 80 °C in 197,63 g gereinigtem Wasser gelöst und die Mischung anschließend auf 40 °C abgekühlt. Dann wurden unter Rühren zunächst 0,75 g Tramadolsaccharinat und anschließend 2 g Xanthangummi zugegeben, eine Stunde nachgerührt und verdunstetes Wasser ersetzt. Nach dem Abkühlen auf Raumtemperatur wurde die Mischung unter Rühren mit 0,625 g Orange-Mandarine Flavor 10888-56 (Givaudan Roure Flavors Ltd. CH 8600 Dübendorf) aromatisiert.

### Beispiel 5:

5 g zerkleinerte Kaugummimasse (Popeye Amural Confections, Yorkville, Illinois, USA) wurden in einer Fantaschale auf eine Temperatur von 30 bis 40 °C erwärmt. In die zähflüssige Kaugummimasse wurden dann mit einem Pistill 150 mg Tramadolsaccharinat eingearbeitet. Die homogene Masse wurde dann in teflonisierten Formen zu Portionen von je 1g portioniert.
Für einen vergleichenden Geschmackstest wurden nach dem selben Verfahren Kaugummis mit der stöchiometrisch gleichen Menge Tramadol (entsprechend 100 mg Tramadolhydrochlorid) hergestellt.

Der Geschmackstest ergab, daß die Kaugummis, die das Tramadolhydrochlorid enthielten, schon nach kurzer Zeit einen unerträglich bitteren Geschmack aufwiesen und nicht weiter gekaut werden konnten. Die Kaugummis, die das Tramadolsaccharinat enthielten, wiesen zu Anfang einen ausgezeichneten Geschmack auf und waren auch nach längerer Kauzeit noch genießbar.

### Beispiel 6:

Zur Herstellung eines Saftes auf wäßriger Grundlage wurden 0,33 g Methylparaben, 0,05 g Propylparaben und 75,0 g Xylitol bei einer Temperatur von 80 °C in 198,37 g gereinigtem Wasser gelöst. Die Mischung wurde auf 40 °C abgekühlt und unter Rühren wurden 0,75 g Tramadolsaccharinat zugegeben. Anschließend wurden 0,25 g Xanthangummi zugegeben, eine Stunde nachgerührt und verdunstetes Wasser ersetzt. Nach dem Abkühlen auf Raumtemperatur wurde die Mischung unter Rühren mit 0,075 g Tutti Frutti 9/008897 (Dragoco Gerberding & Co. AG, 37603 Holzminden) aromatisiert.

### Beispiel 7:

Zur Herstellung eines Saftes auf öliger Grundlage wurden 0,33 g Methylparaben und 0,05 g Propylparaben bei einer Temperatur von 80 °C in 209,88 g mit C₈₋₁₀ gesättigten Fettsäuren verestertem Glycerin gelöst. Die Mischung wurde auf 25 °C abgekühlt und unter Rühren wurden 37,5 g gemahlenes Xylitol, 1,25 g hochdisperses Siliciumdioxid und 0,75 g Tramadolsaccharinat suspendiert. Anschließend wurde die Mischung unter Rühren mit 0,0125 g Blutorange 9/028658 (Dragoco Gerberding & Co. AG, 37603 Holzminden) aromatisiert.

### Beispiel 8:

Zur Herstellung eines Sublingualsprays wurden zunächst 0,33 g Methylparaben, 0,05 g Propylparaben und 75,0 g Xylitol bei einer Temperatur von 80°C in 197,37 g destilliertem Wasser gelöst. Die Lösung wurde auf 40°C abgekühlt und mit 0,75 g Tramadolsaccharinat versetzt. Anschließend wurden 0,15 g Xanthangummi zugegeben, eine Stunde nachgerührt und verdunstetes Wasser ersetzt. Die Lösung wurde auf 25°C abgekühlt und mit 0,75 g Grapefruit 14391786 (IFF, International Flavors & Fragrances GmbH, 46446 Emmerich) aromatisiert.

## Patentansprüche

1. Pharmazeutisches Salz aus Tramadol und aus wenigstens einem Zuckeraustauschstoff.

2. Pharmazeutisches Salz nach Anspruch 1, **dadurch gekennzeichnet, daß** die Löslichkeit des Salzes in Wasser und/oder in wäßrigen Körperflüssigkeiten ≤ 100 mg/ml, vorzugsweise ≤ 30 mg/ml, besonders bevorzugt ≤ 10 mg/ml beträgt.

3. Pharmazeutisches Salz nach Anspruch 1 oder 2, **dadurch gekennzeichnet, daß** der Zuckeraustauschstoff Saccharin, Cyclamat oder Acesulfam ist.

4. Arzneimittel enthaltend wenigstens ein Tramadol-Salz nach einem der Ansprüche 1 bis 3 und gegebenenfalls weitere Wirkstoffe und/oder Hilfsstoffe.

5. Arzneimittel nach Anspruch 4 zur Behandlung/Bekämpfung bei/von Schmerzen, Harninkontinenz, Husten, inflammatorischen und allergischen Reaktionen, Depressionen, Drogen- und/oder Alkoholmißbrauch, Gastritis, Diarrhoe, cardiovaskulären Erkrankungen, Atemwegserkrankungen, seelischen Erkrankungen und/oder Epilepsie.

6. Verwendung wenigstens eines Tramadol-Salzes nach einem der Ansprüche 1 bis 3 zur Herstellung eines Arzneimittels zur Bekämpfung von Schmerzen.

7. Verwendung wenigstens eines Tramadol-Salzes nach einem der Ansprüche 1 bis 3 zur Herstellung eines Arzneimittels zur Behandlung von Harninkontinenz.

8. Verwendung wenigstens eines Tramadol-Salzes nach einem der Ansprüche 1 bis 3 zur Herstellung eines Arzneimittels zur Behandlung von Husten.

9. Verwendung wenigstens eines Tramadol-Salzes nach einem der Ansprüche 1 bis 3 zur Herstellung eines Arzneimittels zur Behandlung von inflammatorischen und allergischen Reaktionen.

10. Verwendung wenigstens eines Tramadol-Salzes nach einem der Ansprüche 1 bis 3 zur Herstellung eines Arzneimittels zur Behandlung bei Depressionen.

11. Verwendung wenigstens eines Tramadol-Salzes nach einem der Ansprüche 1 bis 3 zur Herstellung eines Arzneimittels zur Behandlung bei Drogen und/oder Alkoholmißbrauch.

12. Verwendung wenigstens eines Tramadol-Salzes nach einem der Ansprüche 1 bis 3 zur Herstellung eines Arzneimittels zur Behandlung von Gastritis.

13. Verwendung wenigstens eines Tramadol-Salzes nach einem der Ansprüche 1 bis 3 zur Herstellung eines Arzneimittels zur Behandlung von Diarrhoe.

14. Verwendung wenigstens eines Tramadol-Salzes nach einem der Ansprüche 1 bis 3 zur Herstellung eines Arzneimittels zur Behandlung von cardiovaskulären Erkrankungen.

15. Verwendung wenigstens eines Tramadol-Salzes nach einem der Ansprüche 1 bis 3 zur Herstellung eines Arzneimittels zur Behandlung von Atemwegserkrankungen.

16. Verwendung wenigstens eines Tramadol-Salzes nach einem der Ansprüche 1 bis 3 zur Herstellung eines Arzneimittels zur Behandlung von seelischen Erkrankungen.

17. Verwendung wenigstens eines Tramadol-Salzes nach einem der Ansprüche 1 bis 3 zur Herstellung eines Arzneimittels zur Behandlung von Epilepsie.

18. Darreichungsformen enthaltend wenigstens ein Tramadol-Salz nach einem der Ansprüche 1 bis 3 und gegebenenfalls weitere Wirkstoffe und/oder Hilfsstoffe.

19. Darreichungsformen nach Anspruch 18, **dadurch gekennzeichnet, daß** sie multipartikulär, vorzugsweise als Granulat, Mikropartikel, Mikrotabletten, Pellets, gegebenenfalls in Kapseln abgefüllt oder als Tabletten, schnell zerfallende Tabletten oder Brausetabletten, oder als zu Tabletten verpreßten Pellets formuliert vorliegen.

20. Darreichungsformen nach Anspruch 19, **dadurch gekennzeichnet, daß** sie wenigstens einen magensaftresistenten Überzug aufweisen.

21. Darreichungsformen nach Anspruch 18, **dadurch gekennzeichnet, daß** sie als Gel, Kaugummi, Saft, vorzugsweise als öliger oder wäßriger Saft, oder als Spray, vorzugsweise als Sublingualspray, formuliert vorliegen.

22. Darreichungsformen nach einem oder mehreren der Ansprüche 18 bis 20, **dadurch gekennzeichnet, daß** sie wenigstens eine retardierende Matrix aufweisen.

23. Darreichungsformen enthaltend wenigstens ein Salz des Tramadols mit einem Zuckeraustauschstoff Cyclamat und/oder Acesulfam **dadurch gekennzeichnet, dass** sie wenigstens einen retardierenden Überzug aufweisen.

## Claims

1. Pharmaceutical salt of tramadol and at least one sugar substitute.

2. Pharmaceutical salt according to claim 1, **characterized in that** the solubility of the salt in water and/or aqueous body fluids is ≤100 mg/ml, preferably ≤30 mg/ml and particularly preferably ≤10 mg/ml.

3. Pharmaceutical salt according to claim 1 or 2, **characterized in that** the sugar substitute is saccharin, cyclamate or acesulfame.

4. Medicament containing at least one tramadol salt according to any one of claims 1 to 3 and optionally other active substances and/or auxiliary substances.

5. Medicament according to claim 4 for the treatment/control of pain, urinary incontinence, coughs, inflammatory and allergic reactions, depression, drug and/or alcohol abuse, gastritis, diarrhoea, cardiovascular disease, respiratory disease, mental illness and/or epilepsy.

6. Use of at least one tramadol salt according to any one of claims 1 to 3 for the preparation of a medicament for the control of pain.

7. Use of at least one tramadol salt according to any one of claims 1 to 3 for the preparation of a medicament for the treatment of urinary incontinence.

8. Use of at least one tramadol salt according to any one of claims 1 to 3 for the preparation of a medicament for the treatment of coughs.

9. Use of at least one tramadol salt according to any one of claims 1 to 3 for the preparation of a medicament for the treatment of inflammatory and allergic reactions.

10. Use of at least one tramadol salt according to any one of claims 1 to 3 for the preparation of a medicament for the treatment of depression.

11. Use of at least one tramadol salt according to any one of claims 1 to 3 for the preparation of a medicament for the treatment of drug and/or alcohol abuse.

12. Use of at least one tramadol salt according to any one of claims 1 to 3 for the preparation of a medicament for the treatment of gastritis.

13. Use of at least one tramadol salt according to any one of claims 1 to 3 for the preparation of a medicament for the treatment of diarrhoea.

14. Use of at least one tramadol salt according to any one of claims 1 to 3 for the preparation of a medicament for the treatment of cardiovascular disease.

15. Use of at least one tramadol salt according to any one of claims 1 to 3 for the preparation of a medicament for the treatment of respiratory disease.

16. Use of at least one tramadol salt according to any one of claims 1 to 3 for the preparation of a medicament for the treatment of mental illness.

17. Use of at least one tramadol salt according to any one of claims 1 to 3 for the preparation of a medicament for the treatment of epilepsy.

18. Forms of administration containing at least one tramadol salt according to any one of claims 1 to 3 and optionally other active substances and/or auxiliary substances.

19. Forms of administration according to claim 18, **characterized in that** they are multiparticulate formulations, preferably in the form of granules, microparticles, microtablets or pellets and optionally filled into capsules or in the form of tablets, rapidly disintegrating tablets or effervescent tablets, or in the form of pellets compressed to tablets.

20. Forms of administration according to claim 19, **characterized in that** they have at least one enteric coating.

21. Forms of administration according to claim 18, **characterized in that** they are formulated as a gel, a chewing gum, a juice, preferably an oil-based or water-based juice, or a spray, preferably a sublingual spray.

22. Forms of administration according to one or more of claims 18 to 20, **characterized in that** they have at least one sustained-release matrix.

23. Forms of administration containing at least one salt of tramadol with cyclamate and/or acesulfame as a sugar substitute, **characterized in that** they have at least one sustained-release coating.

## Revendications

1. Sel pharmaceutique formé de tramadol et d'au moins un produit de remplacement du sucre.

2. Sel pharmaceutique suivant la revendication 1, **caractérisé en ce que** sa solubilité dans l'eau et/ou dans des liquides corporels aqueux est inférieure ou égale à 100 mg/ml, avantageusement inférieure à 30 mg/ml et notamment inférieure à 10 mg/ml.

3. Sel pharmaceutique suivant la revendication 1 ou 2, **caractérisé en ce que** le produit de remplacement du sucre est la saccharine, le cyclamate ou l'acésulfam.

4. Médicament contenant au moins un sel de tramadol suivant l'une des revendications 1 à 3 et, le cas échéant, d'autres substances actives et/ou des substances auxiliaires.

5. Médicament suivant la revendication 4, destiné à traiter/combattre des douleurs, l'incontinence urinaire, la toux, des réactions inflammatoires et allergiques, des dépressions, l'abus de drogues et/ou d'alcool, la gastrite, la diarrhée, des maladies cardio-vasculaires, des maladies des voies respiratoires, des maladies psychiques et/ou l'épilepsie.

6. Utilisation d'au moins un sel de tramadol suivant l'une des revendications 1 à 3 pour la préparation d'un médicament destiné à combattre des douleurs.

7. Utilisation d'au moins un sel de tramadol suivant l'une des revendications 1 à 3 pour la préparation d'un médicament destiné au traitement de l'incontinence urinaire.

8. Utilisation d'au moins un sel de tramadol suivant l'une des revendications 1 à 3 pour la préparation d'un médicament destiné au traitement de la toux.

9. Utilisation d'au moins un sel de tramadol suivant l'une des revendications 1 à 3 pour la préparation d'un médicament destiné au traitement de réactions inflammatoires et allergiques.

10. Utilisation d'au moins un sel de tramadol suivant l'une des revendications 1 à 3 pour la préparation d'un médicament destiné au traitement en cas de dépressions.

11. Utilisation d'au moins un sel de tramadol suivant l'une des revendications 1 à 3 pour la préparation d'un médicament destiné au traitement en cas d'abus de drogues et/ou d'alcool.

12. Utilisation d'au moins un sel de tramadol suivant l'une des revendications 1 à 3 pour la préparation d'un médicament destiné au traitement de la gastrite.

13. Utilisation d'au moins un sel de tramadol suivant l'une des revendications 1 à 3 pour la préparation d'un médicament destiné au traitement de la diarrhée.

14. Utilisation d'au moins un sel de tramadol suivant l'une des revendications 1 à 3 pour la préparation d'un médicament destiné au traitement de maladies cardio-vasculaires.

15. Utilisation d'au moins un sel de tramadol suivant l'une des revendications 1 à 3 pour la préparation d'un médicament destiné au traitement de maladies des voies respiratoires.

16. Utilisation d'au moins un sel de tramadol suivant l'une des revendications 1 à 3 pour la préparation d'un médicament destiné au traitement de maladies psychiques.

17. Utilisation d'au moins un sel de tramadol suivant l'une des revendications 1 à 3 pour la préparation d'un médicament destiné au traitement de l'épilepsie.

18. Formes d'administration contenant au moins un sel de tramadol suivant l'une des revendications 1 à 3 et, le cas échéant, d'autres substances actives et/ou des substances auxiliaires.

19. Formes d'administration suivant la revendication 18, **caractérisées en ce qu'**elles ont une formulation multiparticulaire, avantageusement sous forme de granulés, de microparticules, de microtablettes, de pellets, éventuellement chargées dans des gélules ou sous forme de comprimés, de comprimés à désintégration rapide ou de comprimés effervescents, ou sous forme de pellets pressés en comprimés.

20. Formes d'administration suivant la revendication 19, **caractérisées en ce qu'**elles portent au moins un revêtement résistant au suc gastrique.

21. Formes d'administration suivant la revendication 18, **caractérisées en ce qu'**elles sont formulées en gel, gommes à mâcher, sirop, avantageusement sirop huileux ou aqueux, ou sous forme de spray, avantageusement de spray sublingual.

22. Formes d'administration suivant l'une ou plusieurs des revendications 18 à 20, **caractérisées en ce qu'**elles présentent au moins une matrice à effet retardateur.

23. Formes d'administration contenant au moins un sel de tramadol et du cyclamate et/ou de l'acésulfam comme produit de remplacement du sucre, **caractérisées en ce qu'**elles portent au moins un revêtement à effet retardateur.
